# EUROPÄISCHE PATENTANMELDUNG

(11) **EP 1 130 027 A1**
(43) Veröffentlichungstag der Anmeldung: **05.09.2001**
(21) Anmeldenummer: 00104114.4
(22) Anmeldetag: 29.02.2000
(51) Int. Cl.: C07K 7/06, C12P 21/02, A61K 38/08, A61P 9/04

(54) **Memno-Peptide, Verfahren zu ihrer Herstellung und Verwendung derselben**

(71) Anmelder: Aventis Pharma Deutschland GmbH, 65929 Frankfurt am Main (DE)
(72) Erfinder: Vertésy, Laszlo, Dr., 65817 Eppstein-Vockenhausen (DE); Kogler, Herbert, Dr., 61479 Glashütten (DE); Markus, Astrid, Dr., 65835 Liederbach (DE); Schiell, Matthias, Dr., 65611 Brechen (DE)

(57) **Zusammenfassung**

Die Erfindung betrifft neue Peptid-Derivate, als Memno-Peptide genannt, der allgemeinen Formel I: 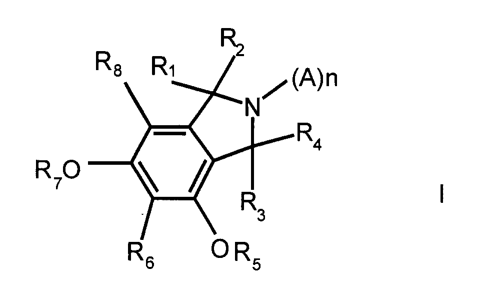 worin R₁, R₂, R₃, R₄, R₅, R₆, R₇, R₈ und (A)n die Bedeutung hierin haben, erhältlich durch Fermentation von *Memnoniella echinata* FH2272, DSM 13195 oder Mutanten und Varianten von dieser, ein Verfahren zur deren Herstellung und die Verwendung der Verbindungen als Arzneimittel, insbesondere gegen Herzinsuffizienzen.

## Beschreibung

Die Erfindung betrifft neue Peptid-Derivate, im folgenden Memno-Peptide genannt, erhältlich durch Fermentation von *Memnoniella echinata* FH2272, DSM 13195 in einem Kulturmedium, ein Verfahren zur Herstellung der Memno-Peptide, die Verwendung der Memno-Peptide als Arzneimittel, insbesondere zur Herstellung eines Arzneimittels zur Behandlung von Herzinsuffizienzen.

Nach wie vor stehen Herz-Kreislauf Erkrankungen in den westlichen Industrieländern als Todesursachen an erster Stelle. Einen nicht unerheblichen Anteil daran stellen Patienten mit der Diagnose Herzinsuffizienz. Unter Herzinsuffizienz versteht man die unzureichende Funktion des Herzens. Das Herz ist nicht imstande, eine den Anforderungen entsprechende Förderleistung zu erbringen. Die Herzinsuffizienz ist ein akutes oder chronisches Unvermögen des Herzens, bei Belastung oder schon in Ruhe den für die Stoffwechsel erforderlichen Blutauswurf aufzubringen bzw. den venösen Rückfluß aufzunehmen. Sie ist der Zustand des Herzens, in dem die Kompensationsmechanismen, wie Herzfrequenz, Schlagvolumen oder Hypertonie, nicht mehr zur Aufrechterhaltung eines normalen Herzzeitvolumens ausreichen. Die Herzinsuffizienz hat vielfältige Ursachen, so vor allem entzündliche und degenerative Myocard-(Herzmuskel-)Veränderungen, koronare Durchblutungsstörungen und der Herzinfarkt. Die Herzinsuffizienz führt zu Veränderungen am peripheren Kreislauf, zu Störung der Atmung, der Nierenfunktion und des Elektrolytstoffwechsels sowie zu verminderter Leistung der Skelettmuskulatur, am Ende führt sie häufig zum Tod.

Die Herzinsuffizienz tritt vor allem im höheren Lebensalter auf. Die Inzidenz beträgt 3 Erkrankungen pro 1000 Einwohner und Jahr bei 35-64jährigen und 10/1000/Jahr in der Altersgruppe von 65-94 Jahren. Die Mortalität steigt bei 75jährigen fast um den Faktor 200 gegenüber der Altersgruppe zwischen 35 und 44 Jahren. Die Mortalitätsrate ist zwischen 1970 und 1983 annähernd konstant geblieben, wie Untersuchungen in den USA ergaben. Für die Bundesrepublik Deutschland sind gleiche Zahlen anzunehmen. Mehr als 50% der Patienten versterben in den ersten fünf Jahren nach Diagnosestellung. Diese statistische Betrachtung zeigt schon allein die große Bedeutung der Herzinsuffizienz für die Bevölkerung, sie belegt aber auch die unzureichenden medikamentösen Behandlungsmöglichkeiten, die heute dem Arzt zu Verfügung stehen.

In dieser Situation wurden neue Konzepte entwickelt, die zu innovativen Kardiaka führen sollen. Die Fähigkeit des Herz- und Skelettmuskeln, sich zu kontrahieren und damit mechanische Arbeit zu leisten, ist (1.) an die Existenz kontraktiler Strukturelemente (Myofibrillen) und (2.) an die Bereitstellung chemischer Energie (ATP) gebunden, die beim Kontraktionsvorgang in mechanische Energie umgewandelt wird. Beim Kontraktionsvorgang kommt es zur Verkürzung der Myofibrillen. Dies wird durch motorische Nervenimpulse eingeleitet, unter deren Wirkung innerhalb weniger Millisekunden Calcium-lonen (Ca²⁺) aus dem extrazellulären Raum in den sarkoplasmatischen Raum eintritt und die Calciumdepots entleert werden. Bei der Myocardinsuffizienz (Herzinsuffizienz) ist die Ca²⁺-Konzentration in Myofibrillen herabgesetzt. Ca²⁺-lonen sind aber für die Aktivierung des kontraktilen Apparats unerläßlich. Bei erhöhter Anforderung wird Ca²⁺ unter Katalyse einer membranständigen Ca²⁺-abhängigen Mg²⁺-ATPase in das Sarcoplasmatische Reticulum (SR) gepumpt: Dieses Enzym wird auch als **S**arko(**E**ndo)plasmatische **R**etikulum **C**a²⁺**A**TPase (SERCA2) bezeichnet. Nach Hydropathieanalyse besteht die Ca²⁺-ATPase aus zehn transmembranären Helices und mehreren extramembranären Schleifen. An der zytosolischen Seite sind Domänen für die Ca²⁺- und ATP-Bindung, für Phosphorylierung und Wechselwirkung mit dem Modulatorprotein Phospholamban (PLB) ausgebildet. Letzteres ist ein Protein-Pentamer, das in der Membran des SR lokalisiert ist und im unphosphorylierten Zustand einen inhibitorischen Einfluß auf SERCA2 ausübt. Unter physiologischer Belastung erfolgt eine Phosphorylierung von PLB, was die Ca²⁺-Affinität von SERCA2a erhöht und damit die Transportrate für Ca²⁺-Ionen in das SR steigert. Die Phosphorylierung von PLB (52 Aminosäuren-Proteins) erfolgt an zwei Aminosäureresten: Serin-16 wird durch die cAMP abhängige Proteinkinase und Threonin an Position 17 durch die Ca²⁺/Calmodulin-abhängige Kinase phosphoryliert. Diese Phosphorylierung bedingt eine Konformationsänderung von PLB gefolgt von einer gesteigerten Affinität von SERCA2 für Ca²⁺. Anti-PLB Antikörper sind in der Lage den PLB-Phosphorilierungseffekt nachzuahmen und beweisen damit die Schlüsselrolle von PLB als Regulator der kontraktilen Aktivität des Herzens (Phospholamban: Protein Structure, Mechanism of Action and Role in Cardiac Function. *H. K. Simmerman and L. R. Jones*, Physiological Reviews; Vol 78, No 4, 921ff, 1998). Daher sollten Aktivatoren von SERCA2 einen günstigen Einfluß bei einer Herzinsuffizienz bewirken.

Es ist überraschend gefunden worden, daß Kulturen des Pilzstammes *Memnoniella echinata* FH 2272, DSM 13195, Naturstoffe enthalten, die günstige Wirkungen auf das Herz und den Kreislauf zu entfalten vermögen. Die isolierten wirksamen Verbindungen, die Memno-Peptide, sind Naturstoffe, die als charakteristische Bestandteile Terpen-bausteine, aber darüber hinaus noch einen sogenannten Polyketidteil sowie einen Stickstoffhaltigen Rest enthalten.

Terpene sind natürlich vorkommenden Verbindungen, die sich formal als Polymerisationsprodukte des Kohlenwasserstoffs Isopren auffassen lassen. Nach Anzahl der Isoprenreste unterscheidet man Monoterpene (C₁₀), Sesquiterpene (C₁₅), Diterpene (C₂₀) usw. Aus den Grundkörpern können durch Substitution, Cyclisierung, Umlagerung, Oxidation usw. eine Vielzahl von Verbindungen gebildet werden, entsprechend sind in der Literatur viele Tausend Terpene beschrieben worden. Auch von den Terpenen abstammenden Stickstoffhaltigen Verbindungen ist berichtet worden, diese werden aber zu den Alkaloiden (z. B. die Gentiana-Alkaloide) gerechnet [Römpp Chemie Lexikon, 9. Auflage, Band 6, Seite 4508 f., Georg Thieme Verlag, Stuttgart/New York, 1992]. Diese Terpene unterscheiden sich jedoch grundsätzlich von den erfindungsgemäßen Memno-Peptiden, die zum Binden des Stickstoffs stets einen Polyketidteil tragen.

Beispiele für weitere, bekannte Stickstoff-haltige Terpene sind:
- Stachybocine [J. Antibiotics, 48, S. 1396 (1995)];
- Stachybotrin [Y. Nozawa et al. J. Antibiotics, 50, S. 635-645, (1997)];
- Spirodihydrobenzo-furanlactame [J. Antibiotics, 49, S. 13, (1996)];
- Nakijiquinones [Tetrahedron , 51, S. 10867-10874 (1995)];
- F1839-A bis J sind stickstoffhaltige Terpene mit Polyketidteilen [ japanisches Patent 061864133 sowie 08283118]. Sie sind Cholesterol-esterase ― Inhibitoren.

Diese Terpenabkömmlige wurden von verschiedenen Stämmen der Gattungen *Memnonielle echinata* und *Stachybotrys* sowie anderen synthetisiert. Sie wurden als Antagonisten des Endothelin-Rezeptors, als Inhibitoren der HIV-1 Protease und der Cholesterol-esterase sowie als Haarwuchsmittel beschrieben. Aus Kulturen des Stammes *Memnoniella echinata,* ATCC 20928, wurde darüber hinaus der Inositol-mono-phosphatase ― Inhibitor L-671,776 isoliert [ Y. K. T. Lam et al. J. Antibiotics, 45, S. 1397-1402, (1992)].

Die erfindungsgemäßen Memno-Peptide weisen ein abweichendes Aktivitätsspektrum auf. Hervorstechendes Merkmal ist ihre aktivierende Wirkung auf SERCA2 und somit auf das insuffiziente Herz.

Die vorliegende Erfindung betrifft somit Verbindungen der folgende allgemeine Formel I: 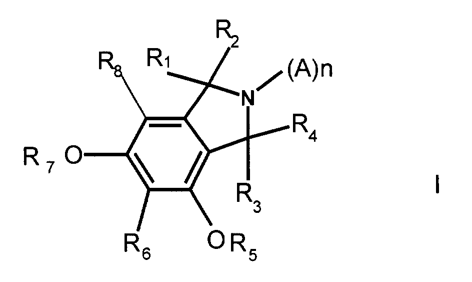 worin bedeuten
- R₁ und R₂: zusammen = O, oder H₂, oder H und OH oder H und O-C₁-C₄-Alkyl;
- R₃ und R₄: zusammen = O, oder H₂, oder H und OH oder H und O-C₁-C₄-Alkyl;
- R8: H, OH, C₁-C₄-Alkyl oder O-C₁-C₄-Alkyl, bevorzugt O-Methyl;
- R₆: einen Rest der Formel II 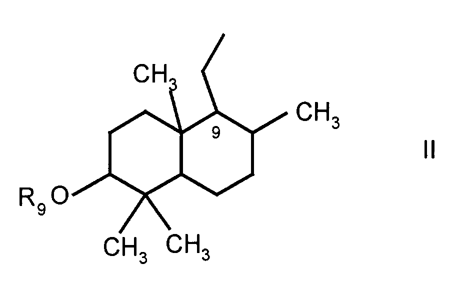 in der R₉ für H oder einen Zucker, glycosidisch gebunden, steht, oder einen Rest der Formel III 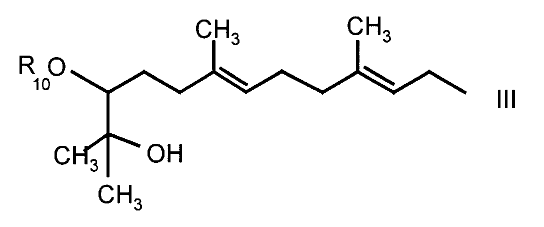 in der R₁₀ für H oder einen Zucker, glycosidisch gebunden, steht, und worin,
wenn R₆ ein Rest der Formel II ist, bedeutet R₅ eine Bindung an das Kohlenstoff-Atom C9 der Formel II und R₇ H, oder R₇ eine Bindung an das Kohlenstoff-Atom C9 der Formel II und R₅ H, und
wenn R₆ ein Rest der Formel III ist, bedeuten R₅ und R₇ H;
- A: eine oder mehrere Aminosäuren, wobei der Stickstoff im Iso-indolring mit dem N-terminalen Aminstickstoff der Aminosäure(n) identisch ist und
- n: die Anzahl der Aminosäure(n) von 1 bis 12;
oder ein physiologisch verträgliches Salz derselben;
mit der Maßgabe daß A eine Aminosäure außer Glu ist, wenn n 1, R₁ und R₂ zusammen =O, R₃ und R₄ zusammen H₂, R₆ einen Rest der Formel II, R₅ eine Bindung an das Kohlenstoff-Atom C9 des Restes der Formel II, R₇ H, R₈ H und R₉ H bedeuten.

In der Formel I steht (A)n vorzugsweise für eine oder mehrere natürliche Aminosäuren ausgewält aus: Gly, Ala, Val, Leu, Ile, Pro, Ser, Thr, Phe, Tyr, Trp, Lys, Arg, Asp, His, Glu, Asn, Gln, Cys und Met. Besonders bevorzugt steht (A)n für eine Peptidkette mit am mindestens 2, insbesondere 10, Aminosäuren.

C₁-C₄-Alkyl bedeutet ein geradkettiges oder verzweigtes Alkyl mit 1 bis 4 C-Atomen, wie z.B. Methyl, Ethyl, i-Propyl und tert. Butyl.

Der Zucker kann einen Aldohexose bedeuten, wie z.B. Mannose.

Die vorliegende Erfindung betrifft weiterhin alle offensichtlichen Abkömmlinge der Verbindungen der Formel I . Abkömmlinge sind Salze, Reduktionsprodukte, Ester, Ether, Acetale sowie Amide und N-alkylierungsprodukte, darüber hinaus alle optische Antipoden, Diastereomere und alle stereomere Formen.

Eine Verbindung der Formel 1 hat vorzugsweise der allgemeinen Formel IV 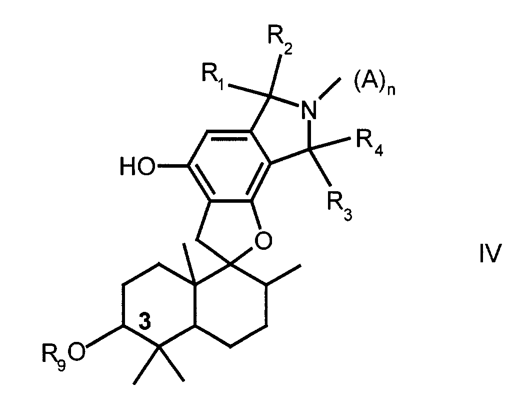 wobei die Reste R₁, R₂, R₃, R₄, R₉ und (A)n die Bedeutung wie oben angegebene haben.

In der Formel IV bedeuten vorzugsweise R₁ und R₂ zusammen =O, R₃ und R₄ zusammen H₂ und R₉ H.

Eine bevorzugte Verbindung ist Memno-Peptid A, C₇₆H₁₀₈N₁₆O₁₈S, der Formel IVa:

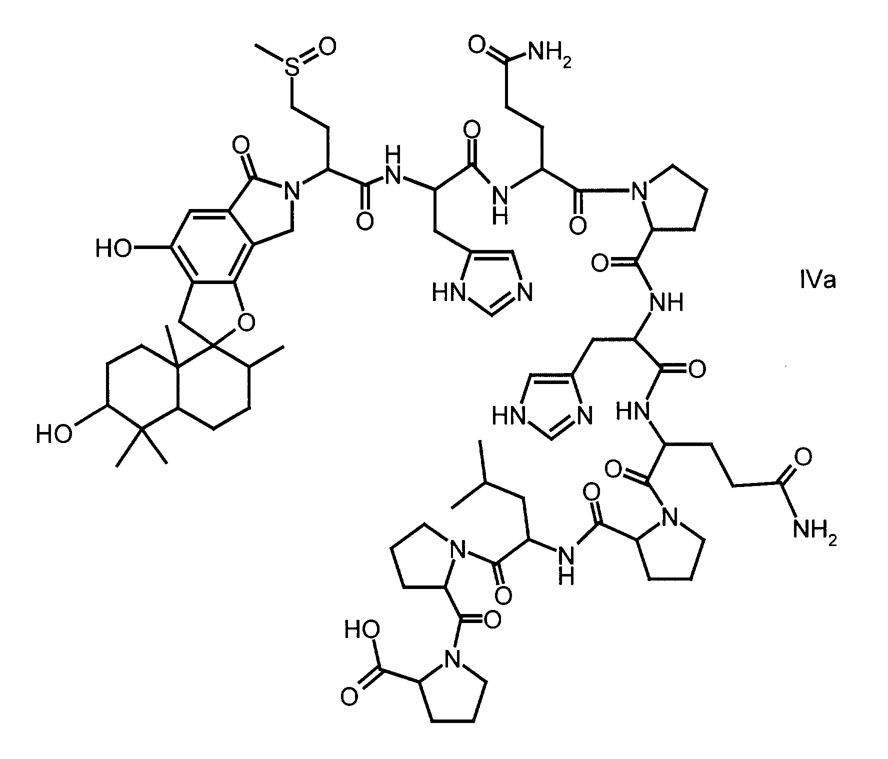

In dieser Formel ist das Dekalin-Gerüst mit den 4 Methyl- und einer Methylen-Gruppe der Terpenteil, das substituierte Isoindol-Ringsystem der Ketid-Teil. Die Aminosäure-Sequenz: Met His Gln Pro His Gln Pro Ile Pro Pro (SEQ ID NO:1) ist ein Teil der Casein-Sequenz. Das Methionin (Met) kann zum Sulfoxid oxidiert sein.

Eine bevorzugte räumliche Form zeigt die Formel IVb: 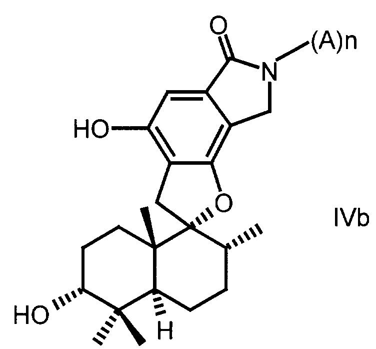 worin (A)n die Bedeutung wie oben angegeben hat.

Die physikalisch-chemischen sowie spektroskopischen Eigenschaften der bevorzugte erfindungsgemäße Verbindung lassen sich wie folgt zusammenfassen:

### Memno-Peptid A

Aussehen: in polaren organischen Lösungsmitteln und in Wasser lösliche, farblose Substanz. Sie ist stabil im neutralen, mild-saurem Milieu .
Summenformel: C₇₆H₁₀₈N₁₆O₁₈S,
Molekulargewicht: 1565,87 Da,
UV-Absorption (λₘₐₓ): 270 nm,
NMR-Daten: siehe Tabelle 1

Die Bezifferung der Kohlenstoffatome und die dazugehörigen NMR-chemischen Verschiebungen sind entsprechend der Zählweise für cyclische Sesqui-Terpene sortiert.

Figur1: Bezifferung eines cyclischen Sesquiterpen-Ketolids.

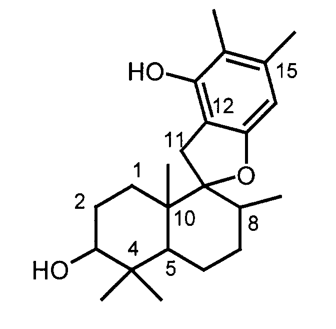

**Tabelle 1:**

| NMR-Daten (chemische Verschiebungen) des Memno-Peptid A in DMSO - d₆ bei 310° K. | | | | | | | |
|---|---|---|---|---|---|---|---|
| | δ-¹³C | m | δ-¹H | m | n_{J_{HH}} | n_{J_{CH}} (10 Hz) | assignment |
| 1 | 15.42 | q | 0.668 | d | 1.802 | 1.802 | Terpen-8-Me |
| 2 | 15.72 | q | 0.978 | s | - | 2.035,1.763 | Terpen-10-Me |
| 3 | 20.37 | t | 1.409 | m | 2.035, 1.538 | 2.035 | Terpen-6 |
| | | | 1.460 | | 2.035, | | |
| 4 | 21.43 | q | 0.873 | d | 1.678 | 1.678, 1.428, 0.890 | Leu⁸-δ |
| 5 | 22.25 | q | 0.827 | s | 0.918 | 0.918, 2.035 | Terpen-4-Me |
| 6 | 22.64 | t | 2,182 | ddt | 4.892, 2.788 2.632 | 4.892, 2.788, 2.632 | Met¹-β |
| | | | 2.307 | ddt | | | |
| | 22.80 | t | 2.19 | ddt | 4.869, 2.757, 2.635 | 4.869, 2.757, 2.635 | |
| | | | 2.31 | ddt | | | |
| 7 | 23.08 | q | 0.890 | d | 1.678 | 1.678, 1.428, 0.873 | Leu⁸-δ' |
| 8 | 23.88 br | d | 1.678 | m | 0.87, 0.88 | | Leu⁸-γ |
| 9 | 23.90 | t | 1.764 | m | 0.962, 1.416, 1.871 | 0.978, 1.416, 3.227 | Terpen-1 |
| | | | 0.962 | m | | | |
| 10 | 24.22 | t | 1.910 | m | 2.144, 4.588, 3.467 | 2.144, 1.771, 4.588, 3.467, 3.689 | Pro⁹-γ |
| 11 | 24.32 | t | 1.863 | m | 2.031, 1.798, 3.625 | 4.356, 3.625, 2.153, 1.792 | Pro⁴-γ |
| 12 | 24.37 | t | 1.927 | m | (2.153), (1.814), 3.624 | 4.388, 3.624, 1.814 | Pro⁷-γ |
| 13 | 24.43 | t | 1.926 | m | 2.144, 1.840, 3.655, 3.538 | 4.224, 3.655, 3.538 | Pro¹⁰-γ |
| 14 | 24.77 | t | 1.840 | m | 1.416, 1.740, 0.962 | - | Terpen-2 |
| | | | 1.416 | m | 1.840, 0.926 | | |
| 15 | 26.51 | t | 1.940 | m | 1.719, 4.484, 2.198 | 2.198 | Gln³-β |
| | | | 1.719 | m | 1.719, 4.484, 2,198 | | |
| 16 | 26.85 | t | 1.927 | m | 1.722, 4.468, 2.166 | 2.166 | Gln⁶-β |
| | | | 1.722 | m | 1.927, 4.468, 2.166 | | |
| 17 | 27.04 | t | 2.976 | dd | 4.598 | - | His⁵-β |
| | br | | 3.067 | dd | | | |
| 18 | 27.09 | t | 2.937 | dd | 4.570 | (4.570) | His²-β |
| | | | 3.067 | dd | | | |
| 19 | 27.50 | t | 2.144 | m | 1.771, 4.578, 1.905, 1.945 | 1.945, 3.689, 3.467, 4.578 | Pro⁹-β |
| | | | 1.771 | m | 2.144, 4.578, 1.905, 1.945 | | |
| 20 | 28.34 | t | 2.144 | m | 1.840, 4.223, 1.926 | 4.223, 3.669, 3.533 | Pro¹⁰-β |
| | | | 1.840 | m | 2.144, 4.223, 1.926 | | |
| 21 | 28.50 | q | 0.918 | s | 0.827 | 0.827 | Terpen-4-Me |
| 22 | 28.86 | t | 1.798 | m | 4.356, 2.031, (1.863) | | Pro⁴-β |
| | | | 2.031 | m | | | |
| 23 | 28.89 | t | 1.814 | m | 2.012, 4.388, (1.927) 1.814, 4.388, (1.927) | 4.388, 3.646 | Pro⁷-β |
| | | | 2.012 | m | | | |
| 24 | 30.63 | t | 1.538 | m | 1.430, 1.460, (1.409), | 0.667 | Terpen-7 |
| | | | | m | (1.802) | | |
| | | | 1.430 | | 1.538, 1.460, 1.802 | | |
| 25 | 30.64 | t | 2.166 | t | 1.927, 1.722 | 1.927, 1.722, 4.466, 6.807 | Gln⁶-γ |
| 26 | 30.65 | t | 2.192 | t | 1.940, 1.719 | 1.940, 1.719, 4.489, 6.789 | Gln³-γ |
| 27 | 31.62 | t | 3.154 | d | 2.792 | 6.598 | Terpen-11 |
| | | | 2.792 | d | 3.154 | | |
| 28 | 36.43 | d | 1.802 | ddq | 1.538, 1.430, 0.667 | 0.667, 2.790, 3.156 | Terpen-8 |
| 29 | 37.22 | s | - | - | - | 0.903, 0.824 | Terpen-4 |
| 30 | 37.73 | q | 2.548 | s | - | 2.682, 2.758 | Met¹-ε |
| | 37.84 | | 2.546 | | | 2.633, 2.783 | |
| 31 | 39.45 | d | 2.035 | dd | 1.409, 1.460 | 0.903, 0.824, 0.971 | Terpen-5 |
| 32 | 40.07 40.03 | t | 1.428 | dt | 4.536, 1.678 | 0.890, 0.873, 4.536 | Leu⁸-β |
| 33 | 41.71 | s | - | - | - | 3.156, 2.790, 0.977, (1.764), 2.035 | Terpen-10 |
| 34 | 44.25 | t | 4.332 | - | - | 4.881 | Terpen-8' |
| 35 | 46.10 | t | 3.669 3.533 | | 1.934 | 4.223, 2.147, 1.934, 1.851 | Pro¹⁰-δ |
| 36 | 46.53 | t | 3.689 3.467 | m m | 1.905,1.936 | 4.588, 2.165, 1.781, 1.936 | Pro⁹-δ |
| 37 | 46.81 | t | 3.624 | m | 1.927 | 1.814, 4.388 | Pro⁷-δ |
| 38 | 46.87 | t | 3.644 | m | 1.863 | - | Pro⁴-δ |
| 39 | 48.56 | d | 4.534 | dt | 7.918,1.428 | 7.918, 1.428, 1.678 | Leu⁸-α |
| 40 | 49.44 | t | 2.682, | ddt | 2.758, 2.162, 2.289 | 4.892, 2.550 | Met¹-γ |
| | | | 2.758 | ddt | 2.682, 2.162, 2.289 2.783, 2.289, 2.162 2.633, 2.289, 2.162 | | |
| | 49.70 | t | 2.633, | ddt | | 4.869, 2.545 | |
| | | | 2.783 | ddt | | | |
| 41 | 50.18 | d | 4.466 | dt | 8.110, 1.927, 1.722 | 2.166 | Gln⁶-α |
| 42 | 50.31 | d | 4.484 | dt | 8.206, 1.935, 1.707 | 2.182 | Gln³-α |
| 43 | 51.37 | d | 4.570 | ddd | 8.135, 2.975, 3.082 | 2.975, 3.082 | His⁵-α |
| 44 | 51.68 | d | 4.590 | ddd | 8.496, 2.934, 3.067 | 2.934, 3.067 | His²-α |
| | | | 4.585 | | 8.506, 2.934, 3.067 | | |
| 45 | 53.30 | d | 4.892 | | 2.162, 2.284 | 2.162 | Met¹-α |
| | 53.62 | | 4.869 | | 2.162, 2.284 | | |
| 46 | 57.33 | d | 4.588 | dd | 2.144, 1.771 | 2.144, 1.771, 1.905, 1.945, 3.684 | Pro⁹-α |
| 47 | 58.31 | d | 4.223 | dd | 2.144, 1.840 | 3.669, 3.553, 1.926, 2.144, (1.840) | Pro¹⁰-α |
| 48 | 59.20 | d | 4.388 | dd | 2.012, 1.814 | 2.012 | Pro⁷-α |
| 49 | 59.44 | d | 4.356 | dd | 2.031, 1.798 | 3.635,1.880 | Pro⁴-α |
| 50 | 73.50 | d | 3.227 | dd | 1.840, 1.416 | 0.903, 0.824 | Terpen-3 |
| | 73.51 | | | | | | |
| 51 | 97.93 97.91 | s | - | - | - | 3.154, 2.792, 0.972, 0.668 | Terpen-9 |
| 52 | 100.90 | d | 6.598 | s | - | - | Terpen-3' |
| | 100.88 | | 6.596 | | | | |
| 53 | 112.62 112.55 | s | - | - | - | 6.593, 4.330 | Terpen-5' |
| 54 | 116.92 116.90 | s | - | - | - | 3.154, 2.792, 6.597 | Terpen-1' |
| 55 | 116.95 | d | 7.230 | s | 8.655 | (3.067), 2.937, 8.665 | His²-ε |
| 56 | 117.26 | d | 7.322 | s | 8.771 | 3.092, 3.000, 8.771 | His⁵-ε |
| 57 | 129.32 | s | - | - | - | 8.783, 7.322, 3.083 2.976, 4.585 | His⁵-γ |
| 58 | 129.63 | s | - | - | - | 7.230, 3.064, 2.937, 4.592 | His²-γ |
| 59 | 133.00 132.98 | s | - | - | - | 4.330 | 1-4' |
| 60 | 133.63 | d | 8.655 | s | 7.230 | 7.230 | His²-δ |
| 61 | 133.68 | d | 8.771 | s | 7.320 | 7.320 | His⁵-δ |
| 62 | 153.60 153.62 | s | - | - | - | 6.597, 3.157, 2.792 | Terpen-2' |
| 63 | 155.73 | s | - | - | - | (6.597), 3.154, 2.792, 4.330 | Terpen-6' |
| 64 | 168.11 168.07 | s | - | - | - | 6.597, 4.330, 4.881 | Terpen-7' |
| 65 | 169.57 | s | - | - | - | 4.588, (4.223) | Pro⁹-CO |
| 66 | 169.59 | s | - | - | - | 8.110, (4.570) | His⁵-CO |
| 67 | 169.69 | s | - | - | - | 4.534, 1.444 | Leu⁸-CO |
| 68 | 169.74 | s | - | - | - | 8.207, 2.937, 4.585 | His²-CO |
| 69 | 169.97 | s | - | - | - | 8.513, 4.869, 2.190 | Met¹-CO |
| | 169.89 | | | | | 8.499, 4.892, 2.182 | |
| 70 | 170.07 | s | - | - | - | 4.471, 1.733, 1.924, 4.356 | Gln³-CO |
| 71 | 170.21 | s | - | - | - | 4.466, 1.722, 1.927, 4.388 | Gln⁶-CO |
| 72 | 170.99 | s | - | - | - | 4.538, 4.388, 2.012, 1.844, 7.918 | Pro⁷-CO |
| 73 | 171.32 | s | - | - | - | 8.138, 4.356, 2.003, 1.805, 4.570 | Pro⁴-CO |
| 74 | 173.10 | s | - | - | - | 4.225, 2.144, 1.840 | Pro¹⁰-CO |
| 75 | 173.80 173.81 | s | - | - | - | 2.19, 1.93, 1.73 | 3-Gln-δ-CO |
| 76 | 173.88 | s | - | - | - | 7.26, 2.19, 1.93, 1.73 | 4-Gln-δ-CO |
| | 173.86 | | | | | | |
| | OH | | 9.75 | br | | NOE : 6.598 | Terpen-2'-OH |
| | NH | | 8.506 | d | 4.585 | NOE: 4.869 /4.692 | His²-NH |
| | | | 8.496 | d | 4.590 | | |
| | NH | | 8.206 | d | 4.484 | NOE : 3.072, 2.948, 4.588 | Gln³-NH |
| | NH | | 8.135 | d | 4.563 | | His⁵-NH |
| | NH | | 8.110 | d | 4.470 | NOE : 4.570, (4.470), 1.727, (1.927) | Gln⁶-NH |
| | NH | | 7.918 | d | 4.534 | NOE : 4.388, 1.827, 1.688, 1.427 | Leu⁸-NH |
| | NH2 | | 7.260 | s | (6.807) | | Gln⁶-NH₂ |
| | | | 6.807 | s | (7.260) | | |
| | NH2 | | 7.230 | s | (6.789) | | Gln³-NH₂ |
| | | | 6.789 | s | (7.230) | | |

Erfindungsgemäß sind die Verbindungen der Formel I erhältlich durch Fermentation des *Memnoniella echinata* FH 2272, DSM 13195, oder eines seiner Varianten oder Mutanten unter geeigneten Bedingungen in einem Kulturmedium, bis sich ein oder mehrere Memno-Peptide der Formel I im Kulturmedium anhäufen, und anschließende Isolierung der Memno-Peptide.

Die Erfindung betrifft daher weiterhin ein Verfahren zur Herstellung einer Verbindung der Formel I, dadurch gekennzeichnet, daß der Mikroorganismus *Memnoniella echinata* FH 2272, DSM 13195, oder einer seiner Varianten oder Mutanten unter geeigneten Bedingungen in einem Kulturmedium fermentiert wird, bis sich ein oder mehrere Memno-Peptide der Formel I im Kulturmedium anhäufen und anschließend aus dem Kulturmedium isoliert werden.

Vorzugsweise wird der Stamm FH 2272, DSM 13195, seine Mutanten und/oder Varianten in einer Nährlösung (auch als Kulturmedium bezeichnet) mit Kohlenstoff und Stickstoffquelle sowie den üblichen anorganischen Salzen fermentiert, bis sich Memno-Peptide in dem Kulturmedium anhäufen. Anschließend werden die Memno-Peptide aus dem Kulturmedium isoliert und gegebenenfalls in die einzelnen aktiven Komponenten aufgetrennt und aufgereinigt.

Als Stickstoffquelle für die Fermentation werden vorzugsweise Aminosäuren und Peptide (A)n, wie oben definiert, verwendet.

Das erfindungsgemäße Verfahren kann für die Fermentation im Labormaßstab (Milliliter- bis Literbereich) und für den industriellen Maßstab (Kubikmetermaßstab) eingesetzt werden.

Eine stark produzierende Kolonie von *Memnoniella echinata* FH 2272, wurde vermehrt. Ein Isolat wurde bei der Deutschen Sammlung von Mikroorganismen und Zellkulturen GmbH, Mascheroder Weg 1B, 3300 Braunschweig, Deutschland, nach den Regeln des Budapester Vertrages am 14. Dezember 1999 unter der folgenden Nummer hinterlegt: *Memnoniella echinata* FH 2272, DSM 13195.

*Memnoniella echinata* FH 2272, DSM 13195, besitzt ein braungrünes Mycel und ist durch die für die Gattung *Memnoniella* characteristischen Conidiophoren gekennzeichnet.

Anstelle des Stammes *Memnoniella echinata* FH 2272, DSM 13195, können auch dessen Mutanten und Varianten eingesetzt werden, die ein oder mehrere Verbindungen der erfindungsgemäßen Memno-Peptide synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden. Als Varianten kommen die nahe verwandte Pilz- Spezies *Stachybotrys,* wie *Stachybotrys atra*, *Stachybotrys chartarum* oder *Stachybotrys complementi* beispielsweise in Betracht.

Das Screening nach Mutanten und Varianten, die ein oder mehrere Verbindungen der erfindungsgemäßen Memno-Peptide synthetisieren, erfolgt nach folgendem Schema:
- Abtrennung des Mycels nach der Fermentation;
- Extraktion der Mycels mit einem organische Lösungsmittel;
- Extraktion der Memno-Peptide aus dem Kulturfiltrat mit Festphasen
- Analytik mittels HPLC, DC oder durch testen der biologischen Wirksamkeit.

Die im folgenden beschriebenen Fermentationsbedingungen gelten für *Memnoniella echinata* FH 2272, das hinterlegte Isolat DSM 13195 sowie Mutanten und Varianten von diesen.

In einer Nährlösung, die eine Kohlenstoffquelle und als Stickstoffquelle Caseinpepton sowie die üblichen anorganischen Salze enthält, produziert *Memnoniella echinata* FH 2272, vorzugsweise DSM 13195, bevorzugt das Memno-Peptid A.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Memno-Peptide verläuft besonders gut in einer Nährlösung, die etwa 0,05 bis 5 %, bevorzugt 0,1 bis 1 % Caseinpepton und 0,2 bis 5 %, bevorzugt 0,5 bis 3 % Glucose, 0,05 bis 1 %, bevorzugt 0,1 bis 0,5 % Cornsteep und Spuren von Kaliumchlorid, Magnesiumsulfat und Eisensulfat enthalten. Die Angaben in Prozent sind jeweils bezogen auf das Gewicht der gesamten Nährlösung.

In dieser Nährlösung bildet *Memnoniella echinata,* vorzugsweise *Memnoniella echinata* FH 2272, DSM 13195, ein Gemisch aus Memno-Peptiden. Je nach Zusammensetzung der Nährlösung kann der mengenmäßige Anteil eines oder mehrerer der erfindungs-gemäßen Memno-Peptide variieren. Außerdem kann durch die Medienzusammensetzung die Synthese einzelner Memno-Peptide gesteuert werden, so daß ein Memno-Peptid gar nicht bzw. in einer Menge unterhalb der Nachweisgrenze vom Mikroorganismus hergestellt wird.

Die Kultivierung des Mikroorganismus erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 37°C, vorzugsweise bei etwa 20 bis 32°C, insbesondere bei 25 bis 30°C durchgeführt werden. Der pH-Bereich sollte zwischen 6 und 8 liegen, vorzugsweise zwischen 6,5 und 7,5. Man kultiviert den Mikroorganismus unter diesen Bedingungen im allgemeinen über einen Zeitraum von 24 bis 300 Stunden, vorzugsweise 36 bis 140 Stunden.

Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Malz-Hefe-Agar oder Potatodextrose-Agar (Standardmedium für Schimmelpilze z.B. von Fa. Difco) wachsen läßt.

Der Fermentationsverlauf kann anhand des pH-Wertes der Kulturen oder des Mycelvolumens sowie durch chromatographische Methoden, wie z.B. Dünnschichtchromatographie oder High Pressure Liquid Chromatography oder Ausprüfen der biologischen Aktivität überwacht werden. Eine erfindungsgemäßen Verbindungen ist sowohl im Mycel als auch im Kulturfiltrat enthalten, der größte Teil befindet sich jedoch im Kulturfiltrat.

Das im folgenden beschriebene Isolierungsverfahren dient zur Aufreinigung der erfindungsgemäßen Memno-Peptide, bevorzugt jedoch für das Memno-Peptid A.

Die Isolierung bzw. Aufreinigung eines erfindungsgemäßen Memno-Peptids aus dem Kulturmedium erfolgt nach bekannten Methoden unter Berücksichtigung der chemischen, physikalischen und biologischen Eigenschaften der Naturstoffe. Zum Testen der Memno-Peptid - Konzentrationen im Kulturmedium oder in den einzelnen Isolierungsstufen kann die Dünnschichtchromatographie, beispielsweise an Kieselgel mit lsopropanol/25%igem NH₃ als Laufmittel oder HPLC verwendet werden. Die Detektion bei der dünnschicht-chromatographischen Auftrennung kann beispielsweise durch Färbereagentien wie Chlorsulfonsäure/Eisessig erfolgen, wobei die Menge der gebildeten Substanz zweckmäßig mit einer Eichlösung verglichen wird.

Zur Isolierung eines erfindungsgemäßen Memno-Peptide wird das Mycel zunächst von der Kulturbrühe nach den üblichen Verfahren abgetrennt und anschließend werden die Memno-Peptide von der Zellmasse mit einem gegebenenfalls mit Wasser mischbaren organischen Lösungsmittel extrahiert. Die organische Lösungsmittelphase enthält die erfindungsgemäßen Naturstoffe, sie wird gegebenenfalls im Vakuum konzentriert und wie unten beschrieben weiter aufgereinigt.

Das Kulturfiltrat wird gegebenenfalls mit dem Konzentrat des Mycel-Extraktes vereinigt und mit einem geeigneten, mit Wasser nicht mischbarem organischen Lösungsmittel, zum Beispiel mit n-Butanol, extrahiert. Die anschließend abgetrennte organische Phase wird ggf. im Vakuum konzentriert. Man kann das Konzentrat zum entfetten des Wertproduktes mit einem unpolaren Lösungsmittel, in dem die erfindungsgemäßen Verbindungen wenig löslich sind, wie zum Beispiel mit Hexan, Petroleum-ether, Diethylether verdünnen. Hierbei präzipitieren die Memno-Peptide, die lipophilen Verunreinigungen bleiben gelöst und werden durch übliche Fest/flüssig-Phasentrennungen entfernt.

Der Niederschlag, der die gesamten Memno-Peptide enthält, wird in 1/30 des ursprünglichen Volumens Wasser/Methanol gelöst. Der Niederschlag geht dabei vollständig in Lösung und wird lyophilisiert. Das Lyophilisat, in der Folge als Rohprodukt bezeichnet, enthält 5 bis 50 % Memno-Peptide und wird für die weitere Isolierung eingesetzt.

Die weitere Aufreinigung eines oder mehrerer erfindungsgemäßen Memno-Peptide erfolgt durch Chromatographie an geeigneten Materialien, vorzugsweise z.B. an Molekularsieben, an Kieselgel, Aluminiumoxid, an lonenaustauschern oder an Adsorberharzen bzw. an Umkehr-Phasen (reversed phase, RP). Mit Hilfe dieser Chromatographie werden die Memno-Peptide getrennt. Die Chromatographie der Memno-Peptide erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, Propanol und Acetonitril, in einer Konzentration von 5 bis 80 % Lösemittel, vorzugsweise 20 bis 50 % Lösemittel oder auch alle gepufferten wässrigen Lösungen, die mit organi-schen Lösemitteln mischbar sind. Die zu verwendeten Puffer sind die gleichen wie oben angegeben.

Die Trennung der Memno-Peptide aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxyd und ähnlichen erfolgen.

Die Chromatographie der Memno-Peptide erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von 0 bis 0,5 M sowie Ameisensäure, Essigsäure, Trifluoressigsäure oder allen handelsüblichen, dem Fachmann bekannten Säuren, vorzugsweise in einer Konzentration von 0 bis 1 %. Besonders bevorzugt wird 0,1 %.

Chromatographiert wird mit einem Gradienten, der mit 100 % Wasser beginnt und mit 100 % Lösemittel endet, vorzugsweise wird ein linearer Gradient von 20 bis 50 % Propanol oder Acetonitril gefahren.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad) oder Fractogel TSK HW 40® (Fa. Merck, Deutschland oder Toso Haas, USA).

Eine weiteres, sehr wirksames Verfahren zur Reinigung der erfindungsgemäßen Verbindungen ist die Anwendung von lonenaustauschern. Zum Beispiel kann das basische Memno-Peptid A an Kationenaustauschern , wie Fractogel® EMD SO₃, sehr vorteihaft isoliert werden. Verwendung finden können Pufferlösungen zwischen pH 5 und 8, vorzugsweise zwischen pH 6 bis 7,5. Die Elution kann z. B. mit einem ansteigenden Salzgradienten erreicht werden. Es kann auch vorteilhaft sein als Lösemittel neben Wasser auch Mischungen von wässrigen Pufferlösungen mit einem organischen Lösungsmittel zu verwenden. Der Anteil des organischen Lösungsmittels liegt zwischen 10% und 90%, vorzugsweise zwischen 30 und 60 %.

Die Reihenfolge der vorgenannten Chromatographien ist umkehrbar.

Ein weiterer, sehr wirksamer Reinigungsschritt für Memno-Peptide ist die Kristallisation. Die Memno-Peptide kristallisieren aus Lösungen in organischen Lösungsmitteln und aus Mischungen von Wasser mit organischen Lösungsmitteln aus. Die Kristallisation wird in an sich bekannter Weise, zum Beispiel durch Konzentrieren oder Abkühlen gesättigter Memno-Peptid-Lösungen, durchgeführt.

Die erfindungsgemäßen Memno-Peptide sind im festen Zustand und in Lösungen im pH-Bereich zwischen 3 und 8, insbesondere 5 und 7, stabil und lassen sich damit in übliche galenische Zubereitungen einarbeiten.

Die Bildung der stickstoffhaltigen Memno-Peptide kann begünstigt werden, indem man der *Memnonielle echinata ―* Kultur die gewünschten Aminosäuren oder Peptide als Vorprodukt hinzufügt. Es ist die Eigenart der *Memnonielle echinata ―* Spezies, daß sie die Aminogruppen der angebotenen Aminosäuren und Peptiden unter Synthese eines Fünfring-Lactams, meistens eines Isoindol-Ringsystems, binden. Diese Bindung geschieht entweder ausgehend von der Aldehyd-Vorstufe im Rahmen einer Oxidation oder von dem Lacton-Oxidatons-Stufe in ringgeöffneter Form oder der cyclische Lactonform. Diese Fähigkeit des *Memnonielle echinata* zur Bindung von Aminen ist völlig überraschend beobachtet worden und kann zur Gewinnung von geschützten, umgewandelten Amin-Derivaten, vorzugsweise von Terpenderivaten von Aminosäuren sowie Peptiden benützt werden.

Eine oder mehrere Verbindungen der erfindungsgemäßen Memno-Peptide eignen sich aufgrund ihrer wertvollen pharmakologischen Eigenschaften zur Anwendung in der Human- oder Tiermedizin als Arzneimittel.

Die vorliegende Erfindung betrifft somit die Verwendung der Verbindung der Formel I oder ein physiologisch verträgliches Salz desselben zur Herstellung eines Kardiakums zur Behandlung oder Propylaxe von Herzinsuffizienz.

Der Wirkmechanismus der Memno-Peptide ist unbekannt, jedoch konnte ein signifikanter Effekt nachgewiesen werden.

Zum Nachweis der Aktivatoren von SERCA2, die den Phosphorylierungs-Effekt von PLB nachahmen, bedient man sich eines colorimetrischen Testes, der die Aktivität der Ca²⁺-ATPase in Hundeherzmikrosomen in Gegenwart von Prüfsubstanzen bestimmt. Der Test wird in 96-well Mikrotiterplatten durchgeführt: Gemessen wird die enzymatische Freisetzung von anorganischem Phosphat aus ATP, das mit Ammoniummolybdat einen blauen Farbstoffkomplex bildet, der bei 620 nm im Spektralphotometer gemessen werden kann.

Der IC₅₀ für das Memno-Peptid A ist 6.5 µM; es ist die Konzentration, die SERCA2 zu 50 % aktiviert.

Die Memno-Peptide haben darüber hinaus eine antimikrobielle Wirkung.
Die vorliegende Erfindung betrifft somit die Verwendung der Verbindung der Formel I oder ein physiologisch verträgliches Salz desselben zur Herstellung eines Arzneimittels zur Behandlung mikrobieller, insbesondere bakterieller, Infektionen.

Vom antimikrobiellen Spektrum zeigt die Tabelle 3 einige minimalen Hemmkonzentrationen (MHK) des Memno-peptids A gegen einige ausgewählte Bakterien.

**Tabelle 3:**

| Stamm | Resistenz gegen: | MHK [µg/ml] |
|---|---|---|
| *Staphylococcus aureus* SG 511 | - | 16 |
| *Staphylococcus aureus* 285 | Penicillin | 16 |
| *Staphylococcus aureus* 503 | Penicillin | 16 |
| *Staphylococcus aureus* FH 1982 | Methicillin | 16 |
| *Staphylococcus aureus* 701E | Methicillin | 16 |
| *Staphylococcus aureus* 707E | Methicillin | 16 |
| *Staphylococcus aureus* 9 Tüb | Ofloxacin | 16 |
| *Staph. epidermidis* ZH 2c | - | 16 |
| *Staph. epidermidis* 763 | Methicillin | > 16 |
| *Staph. epidermidis* 5747IIW | Methicillin | 32 |
| *Staph. epidermidis 291* | Ofloxacin | 8 |
| *Staph. epidermidis* 799 | Ofloxacin | 8 |
| *Enterococcus faecium* Md8B | - | 8 |
| *Enterococcus faecium* VR1 | Vancomycin | > 64 |
| *Enterococcus faecium* VR2 | Vancomycin | > 64 |
| *Streptococcus pyogenes* VR3 | Vancomycin | > 64 |
| *Streptococcus pyogenes* 308A | - | 8 |
| *Streptococcus pyogenes* 77A | - | 4 |

Neben der antibakteriellen Wirkung besitzen die erfindungsgemäßen Verbindungen schwache antimykotische, d. h. pilzhemmende Eigenschaften, zum Beispiel gegen *Candida albicans.*

Darüber hinaus besitzen die erfindungsgemäßen Substanzen eine gewisse günstige Hemmwirkung auf die Glucose-6-Phosphat - Translocase (G-6-P ― TL), einem Enzym der für den Glucose-Stoffwechsel und damit für die Behandlung des Diabetes mellitus von Bedeutung ist. Insbesondere die Verbindung Memno-Peptid A zeigt selektive Aktivitäten gegen die G-6-P ― TL, sie hemmt aber nicht das Coenzym G-6-P ― Phosphatase.

Die vorliegende Erfindung betrifft somit die Verwendung der Verbindung der Formel I oder ein physiologisch verträgliches Salz desselben zur Herstellung eines Arzneimittels zur Therapie der Diabetes mellitus.

Die Erfindung betrifft auch pharmazeutische Zubereitungen einer oder mehrerer Verbindungen der erfindungsgemäßen Memno-Peptide.

Ein oder mehrere Verbindungen der erfindungsgemäßen Memno-Peptide, bevorzugt ein oder mehrere Verbindungen der Memno-Peptide, können grundsätzlich als solche in Substanz verabreicht werden. Bevorzugt ist die Verwendung in Mischung mit geeigneten Hilfsstoffen oder Trägermaterial. Als Trägermaterial können bei Arzneimitteln die üblichen und pharmakologisch verträglichen Trägermaterialien und/oder Hilfsstoffe verwendet werden.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Memno-Peptide enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere Verbindungen der erfindungsgemäßen Memno-Peptide mit üblichen Träger- sowie gegebenenfalls Zusatz- und/oder Hilfstoffe in die bzw. eine geeignete Darreichungsform bringt.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1. Herstellung einer Glycerinkultur von Memnoniella echinata FH 2272, DSM 13195,

100 ml Nährlösung (Malzextrakt 2,0%, Hefeextract 0,2 %, Glucose 1,0 % (NH₄)₂ HPO₄ 0,05 %, pH 6,0) in einem sterilen 300 ml Erlenmeyerkolben werden mit dem Stamm *Memnoniella echinata* FH 2272, DSM 13195, beimpft und 7 Tage bei 25° C und 140 UpM auf einer rotierenden Schüttelmaschine inkubiert. 1,5 ml dieser Kultur werden anschließend mit 2,5 ml 80 %igem Glycerin verdünnt und bei -20°C gelagert.

### Beispiel 2. Herstellung einer Kultur bzw. einer Vorkultur im Erlenmeyerkolben von Memnoniella echinata FH 2272, DSM 13195.

Ein steriler 300 ml Erlenmeyerkolben mit 100 ml der folgende Nährlösung 10 g/l Glucose, 5 g/l Casein-Pepton, 1,7 g/l Cornsteep flüssig, und 7 ml Spurenelementlösung (10 g/l KCI, 10 g/l MgSO₄ × 7 H₂O, 3,6 g/l FeSO₄ x 7 H₂O und 6 g/l MgSO₄ H₂O) wird mit einer auf einem Schrägröhrchen (gleiche Nährlösung, jedoch mit 2 % Agar) gewachsenen Kultur oder mit 1 ml einer Glycerinkultur (s. Beispiel 1) beimpft und auf einer Schüttelmaschine bei 180 UpM und 30 C inkubiert. Die maximale Produktion einer oder mehrerer Verbindungen der erfindungsgemäßen Memno-Peptide ist nach ca. 120 Stunden erreicht. Zum Animpfen von 10 und 200 I Fermentern genügt eine 48 bis 96 Std. alte Submerskultur (Animpfmenge ca. 10 %) aus der gleichen Nährlösung.

### Beispiel 3. Herstellung der Memno-Peptide.

Ein 30 I Fermenter wird unter folgenden Bedingungen betrieben:

| | |
|---|---|
| Nährmedium | 10 g/l Glucose |
| | 0,5 g/l Casein-Pepton |
| | 1,7 g/l Cornsteep flüssig |
| | 7 ml Spurenelementlösung |
| | pH 6,5 (vor der Sterilisation) |
| Spurenelementlösung | KCI 10 g/l, MgSO₄ x 7 H₂O 10 g/l, FeSO₄ x 7 H₂O 3,6 g/l und MnSO₄ x H₂O 6 g/l |
| Inkubationszeit | 45 Stunden |
| Inkubationstemperatur | 28° C |
| Rührergeschwindigkeit | 300 UpM |
| Belüftung | 15 l Min⁻¹ |

Durch wiederholte Zugabe von ethanolischer Polyollösung kann die Schaumbildung unterdrückt werden. Das Produktionsmaximum wird nach ca. 35 bis 70 Stunden erreicht.

### Beispiel 4. Isolierung des Memno-Peptid - Gemisches aus der Kulturlösung des Memnoniella echinata FH 2272, DSM 13195.

Nach Beendigung der Fermentation von *Memnoniella echinata* FH 2272, DSM 13195, wird die Kulturbrühe der Fermenter, gewonnen nach Beispiel 3, (200 Liter) unter Zusatz von ca. 2 % Filterhilfsmittel (z.B. Celite®) filtriert und die Zellmasse (22 Liter) mit 66 Liter Methanol extrahiert. Die Wertstoff-haltige, methanolische Lösung wird durch Filtration vom Mycel befreit und im Vakuum konzentriert. Das Konzentrat wird mit Wasser verdünnt und gemeinsam mit dem Kulturfiltrat (180 Liter) auf eine vorbereitete, 17 Liter MCI GEL, CHP20P-Säule aufgetragen. Eluiert wird mit einem Gradienten von Wasser nach 60% Propanol-2 in Wasser. Der Säulendurchfluß (25 Liter pro Stunde) wird fraktioniert aufgefangen (je 10 Liter) und die Memno-Peptide enthantenden Fraktionen (von 25% bis 30 % Propanol-2) zusammengefaßt. Konzentrieren im Vakuum ergeben 20 Liter einer braunen Lösung. In eine Säule (125 mm x 500 mm) werden 6 Liter, mit Kalium-Phosphat-Puffer bei pH 7 äquilibrierter Kationenaustauscher, Fractogel® EMD SO₃⁻, gefüllt. Nach Beladen des lonenaustauschers mit 20 Liter des oben beschriebenen Konzentrates wird mit einem Gradient von 10 mM Kalium-Phosphat-Puffer, pH 7, nach 1 M NaCI in 10 mM Kalium-Phosphat-Puffer, pH 7 in Wasser/ Methanol (1:1) eluiert. Der Säulen-Durchbruch, d. h. das nicht gebundene Material enthält die neutralen Memno-Peptide (49 g). Der Säulen-Durchfluß beträgt 12 Liter pro Stunde, er wird während der Gradienten-Elution 1 Liter-Portionen fraktioniert aufgefangen . Mit 0.75 M NaCI (Fraktionen 31 und 32) gewinnt man das Memno-Peptid A. Die Fraktionen 31 und 32 werden zusammengefaßt und im Vakuum auf etwa 500 ml konzentriert.

### Beispiel 5: Anreicherung des Memno-Peptids A durch Gel-Chromatographie.

8 g des nach Beispiel 4 gewonnenen Produktes werden auf eine 3,9 Liter fassende mit Fractogel® TSK HW-40 s gefüllte Säule (Breite x Höhe = 10 cm x 50 cm) aufgetragen. Das Laufmittel: Methanol-Wasser (1:1) wird mit einer Flußrate von 20 ml pro Minute über die Säule gepumpt und der Säulenausfluß fraktioniert (20 ml) aufgefangen. Hauptsächlich in den Fraktionen 75 bis 85 befinden sich die Memno-Peptide A. Sie werden zusammengefaßt und im Vakuum vom Methanol befreit. Sie ergeben 0.9 g Wirkstoff-Gemisch.

### Beispiel 6. HPLC System zum Nachweis der Memno-Peptide.

Das unten beschriebene System erlaubt die Reinheitsprüfung sowie Trennung und Quantifiziertung der Memnoterpene, zum Beispiel im Rohgemisch bzw. im Kulturfiltraten.

| | |
|---|---|
| Laufmittel | 0,1 % Trifluoressigsäure in 32 % Acetonitril. |
| Säule | Nucleosil 100C₁₈AB 250/4, Macherey-Nagel. |
| Fluß | 1,0 ml/Min |
| Detektion | Ultravioletlicht-Absoption bei 210 nm. |

Unter den angegebenen Bedingungen hat das Memno-Peptid A folgende Retentionszeit

| | |
|---|---|
| Memno-Peptid A | 7,0 Minuten, |

### Beispiel 7. Reinigung des Memno-Peptids A.

500 ml Lösung des nach Beispiel 5 isolierten und angereicherten Memno-Peptids A werden auf 500 ml Nucleosil® 100-7 C₁₈AB - Säule aufgetragen und mit einem Gradienten von 25 bis 50 % Acetonitrl in 0.05 % Trifluoressigsäure/Wasser chromatographiert. Der Durchfluß des Elutionsmittels beträgt 50 ml pro Minute, die Fraktionsgröße 50 ml. In den Fraktionen 71 bis 88 befindet sich das Memno-Peptid A. Die wiederholte Reinigung der zusammengefaßten Fraktionen mit konstanter Lösungsmittelkonzentration von 28 % Acetonitril in 0.05 % Trifluoressigsäure ergibt > 95 % reines Memno-Peptid C nach Gefriertrocknung (100 mg).

### Charakterisierung des Memno-Peptid A:

10 µg Memno-Peptid A werden in konstant-siedender Salzsäure hydrolysiert und auf einem Aminosäuren-Analysator untersucht. Es werden folgende übliche Aminosäuren gefunden:

| | |
|---|---|
| Glutaminsäure | 11 nMol |
| Prolin | 22 nMol |
| Histidin | 10 nMol |
| Leucin | 5,6 nMol |
| Methionin-oxid | 5,5 nMol |

Ultraviolett Absorption: λₘₐₓ bei 269 nm, 305 nm (Schulter).

Das hochauflösende FAB Massenspektrum zeigt ein intensives MH⁺ bei m/z 1565.7819 Da, in guter Übereinstimmung mit der berechneten Masse (für C₇₆H₁₀₉N₁₆O₁₈S, mono-isotopisch) von 1565.7827 Da. Die MS/MS ― Fragmentierung entspricht der Formel IVa.

## Patentansprüche

1. Verbindung der allgemeinen Formel I 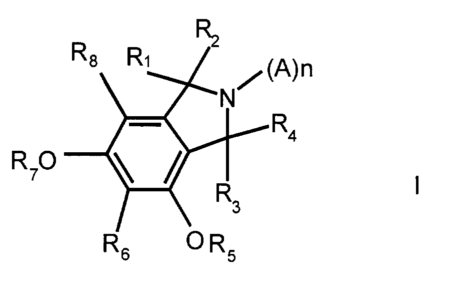 worin bedeuten
R₁ und R₂ zusammen = O, oder H₂, oder H und OH oder H und O-C₁-C₄-Alkyl;
R₃ und R₄ zusammen = O, oder H₂, oder H und OH oder H und O-C₁-C₄-Alkyl;
R₈ H, OH, C₁-C₄-Alkyl oder O-C₁-C₄-Alkyl;
R₆ einen Rest der Formel II 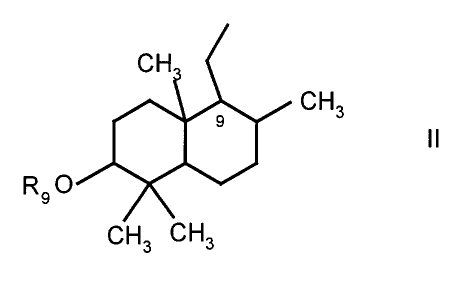 in der R₉ für H oder einen Zucker, glycosidisch gebunden, steht, oder einen Rest der Formel III 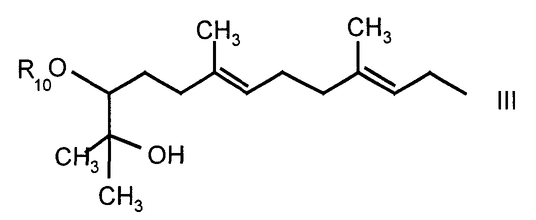 in der R₁₀ für H oder einen Zucker, glycosidisch gebunden, steht, und worin,
wenn R₆ ein Reste der Formel II ist, bedeutet R₅ eine Bindung an das Kohlenstoff-Atom C9 der Formel II und R₇ H oder R₇ eine Bindung an das Kohlenstoff-Atom C9 der Formel II und R₅ H; und wenn R₆ ein Reste der Formel III ist, bedeuten R₅ und R₇ H;
A eine oder mehrere Aminosäuren, wobei der Stickstoff im Iso-indolring mit dem N-terminalen Aminstickstoff der Aminosäure(n) identisch ist und
n die Anzahl der Aminosäure(n) von 1 bis 12;
oder ein physiologisch verträgliches Salz derselben;
mit der Maßgabe daß A eine Aminosäure außer Glu ist, wenn n 1, R₁ und R₂ zusammen =O, R₃ und R₄ zusammen H₂, R₆ ein Rest der Formel II, R₅ eine Bindung an das Kohlenstoff-Atom C9 des Restes der Formel II, R₇ H, R₈ H und R₉ H bedeuten.

2. Verbindung der Formel I oder ein physiologisch verträgliches Salz derselben gemäß Anspruch 1 der allgemeinen Formel IV 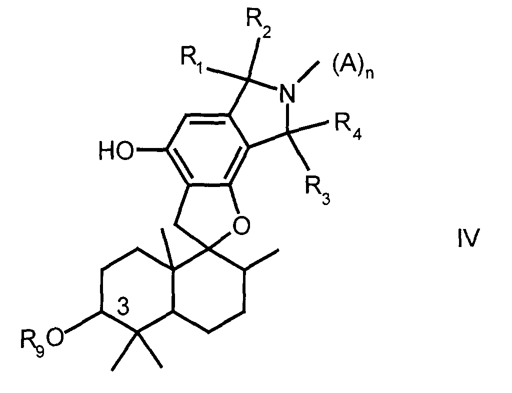 wobei die Reste R₁, R₂, R₃, R₄, R₉ und (A)n die Bedeutung wie oben angegeben haben.

3. Verbindung oder ein physiologisch verträgliches Salz derselben, gemäß Anspruch 2 worin R₁ und R₂ zusammen =O, R₃ und R₄ zusammen H₂ und R₉ H bedeuten.

4. Verbindung oder ein physiologisch verträgliches Salz derselben, gemäß Anspruch 1 der Formel V 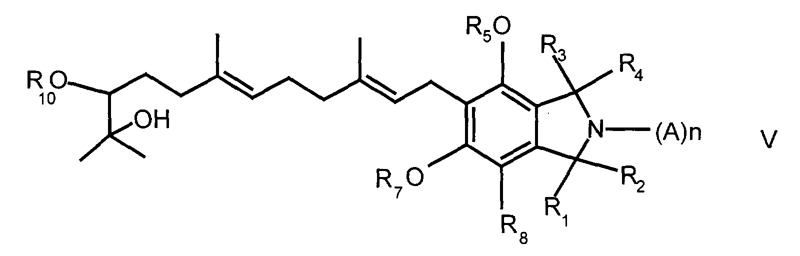 worin R₁, R₂, R₃, R₄, R₅, R₇, R₈ und R₁₀ die Bedeutung, wie oben angegeben, haben.

5. Verbindung der Formel I oder ein physiologisch verträgliches Salz derselben, gemäß einem oder mehreren der Ansprüche 1 bis 4, worin (A)n für eine Peptidkette mit 2 bis 12 natürlichen Aminosäure-Resten steht.

6. Verbindung der Formel I oder ein physiologisch verträgliches Salz derselben gemäß einem oder mehreren der Ansprüche 1 bis 5, worin (A)n für die Peptidkette: Met-His-Gln-Pro-His-Gln-Pro-lle-Pro-Pro (SEQ ID NO: 1) steht in der das Met zum Sulfoxid oxidiert sein kann.

7. Verbindung der Formel I oder ein physiologisch verträgliches Salz derselben gemäß einem oder mehreren der Ansprüche 1 bis 6, worin R₉ oder R₁₀ eine Aldohexose bedeutet.

8. Verbindung der Formel I gemäß einem oder mehreren der Ansprüche 1 bis 7 erhältlich durch Fermentation von *Memnoniella echinata* FH 2272, DSM 13195 , oder einer seiner Varianten oder Mutanten unter geeigneten Bedingungen in einem Kulturmedium, bis sich eine Verbindung der Formel I in dem Kulturmedium anhäuft, anschließende Isolierung der Verbindung und gegebenenfalls Umwandlung in chemische Derivate, sowie deren physiologisch verträglichen Salze.

9. Verfahren zur Herstellung einer Verbindung der Formel I oder eines physiologisch verträglichen Salzes derselben gemäß einem oder mehreren der Ansprüche 1 bis 8, umfassend die Fermentation von *Memnoniella echinata* FH 2272, DSM 13195, oder einer seiner Varianten oder Mutanten unter geeigneten Bedingungen in einem Kulturmedium bis sich eine Verbindung der Formel I in dem Kulturmedium anhäuft und anschließende Isolierung der Verbindung und gegebenenfalls Umwandlung in chemische Derivate und/oder physiologisch verträglichen Salze.

10. Verbindung der Formel I oder ein physiologisch verträgliches Salz derselben gemäß einem oder mehreren der Ansprüche 1 bis 8 zur Verwendung als Arzneimittel.

11. Verwendung eine Verbindung der Formel I oder ein physiologisch verträgliches Salz derselben gemäß einem oder mehreren der Ansprüche 1 bis 8 zur Herstellung eines Arzneimittels zur Behandlung oder Propylaxe der Herzinsuffizienz.

12. Verwendung eine Verbindung der Formel I oder ein physiologisch verträgliches Salz derselben gemäß einem oder mehreren der Ansprüche 1 bis 8 zur Behandlung von Krankheiten, bei denen die Herzinsuffizienz eine primäre oder sekundäre Ursache darstellt, und somit ihre Verwendung als Mittel gegen Herz- und Kreislaufschwächen.

13. Verwendung eine Verbindung der Formel I oder ein physiologisch verträgliches Salz derselben gemäß einem oder mehreren der Ansprüche 1 bis 8 zur Herstellung eines Arzneimittels zur Therapie des Diabetes mellitus.

14. Verwendung eine Verbindung der Formel I oder ein physiologisch verträgliches Salz derselben gemäß einem oder mehreren der Ansprüche 1 bis 8 zur Herstellung eines Arzneimittels zur Bekämpfung mikrobieller Infektionen.

15. Pharmazeutische Zubereitung, enthaltend mindestes eine Verbindung der Formel I und/oder mindestens ein physiologisch verträgliches Salz derselben gemäß einem oder mehreren der Ansprüche 1 bis 8.

16. Verfahren zur Herstellung eines pharmazeutischen Präparats, **dadurch gekennzeichnet**, daß mindestens eine Verbindung der Formel I und/oder mindestens ein physiologisch verträgliches Salz derselben gemäß einem oder mehreren der Ansprüche 1 bis 8, gegebenenfalls mit pharmazeutisch üblichen Trägerstoffen oder Verdünnungsmitteln in eine pharmazeutisch geeignete Verabreichungsform gebracht wird.
